# EUROPEAN PATENT SPECIFICATION

(11) **EP 2 836 603 B1**
(45) Date of publication and mention of the grant of the patent: **06.06.2018**
(21) Application number: 13775998.1
(22) Date of filing: 04.04.2013
(51) Int. Cl.: C12P 19/34

(54) **SYNTHETIC NUCLEIC ACIDS FOR POLYMERIZATION REACTIONS**
SYNTHETISCHE NUKLEINSÄUREN FÜR POLYMERISATIONSREAKTIONEN
ACIDES NUCLÉIQUES SYNTHÉTIQUES DESTINÉS À DES RÉACTIONS DE POLYMÉRISATION

(30) Priority: 12.04.2012 US 201261623110 P
(43) Date of publication of application: 18.02.2015
(73) Proprietor: NEW ENGLAND BIOLABS, INC., Ipswich, MA 01938-2723 (US)
(72) Inventor: JOHNSON, Donald, Brookline, MA (US); EVANS, Thomas, C., Topsfield, MA 01983 (US); GREENOUGH, Lucia, Ipswich, MA 01938 (US); ONG, Jennifer, Salem, MA 01970 (US)
(74) Representative: Griffin, Philippa Jane
(86) International application number: PCT/US2013/035217
(87) International publication number: WO 2013/154898

(56) References cited:
- WO-A1-01/02559
- WO-A1-2013/033528
- WO-A1-2015/091720
- WO-A2-2007/038422
- US-A- 5 693 502
- US-A1- 2003 180 737
- US-A1- 2007 141 591
- US-A1- 2009 098 539
- US-A1- 2011 262 898
- US-A1- 2013 230 887
- US-B1- 6 238 865
- MONNARD P A ET AL: "Metal-ion catalyzed polymerization in the eutectic phase in water-ice: A possible approach to template-directed RNA polymerization", JOURNAL OF INORGANIC BIOCHEMISTRY, ELSEVIER INC, US, vol. 102, no. 5-6, 1 May 2008 (2008-05-01) , pages 1104-1111, XP022611986, ISSN: 0162-0134, DOI: 10.1016/J.JINORGBIO.2008.01.026 [retrieved on 2008-02-01]
- VO M N ET AL: "Mechanistic Studies of Mini-TAR RNA/DNA Annealing in the Absence and Presence of HIV-1 Nucleocapsid Protein", JOURNAL OF MOLECULAR BIOLOGY, ACADEMIC PRESS, UNITED KINGDOM, vol. 363, no. 1, 13 October 2006 (2006-10-13), pages 244-261, XP024951465, ISSN: 0022-2836, DOI: 10.1016/J.JMB.2006.08.039 [retrieved on 2006-10-13]
- ANDO YOSHINARI ET AL: "Two-step cleavage of hairpin RNA with 5' overhangs by human DICER", BMC MOLECULAR BIOLOGY, BIOMED CENTRAL LTD, GB, vol. 12, no. 1, 9 February 2011 (2011-02-09), page 6, XP021086661, ISSN: 1471-2199, DOI: 10.1186/1471-2199-12-6
- MEGHAN MITCHELL ET AL: "Structural basis for telomerase catalytic subunit TERT binding to RNA template and telomeric DNA", NATURE STRUCTURAL & MOLECULAR BIOLOGY, vol. 17, no. 4, 28 March 2010 (2010-03-28) , pages 513-518, XP055056181, ISSN: 1545-9993, DOI: 10.1038/nsmb.1777
- KAINZ PETER ET AL: "Specificity-enhanced hot-start PCR: Addition of double-stranded DNA fragments adapted to the annealing temperature", BIOTECHNIQUES, INFORMA HEALTHCARE, US, vol. 28, no. 2, 1 February 2000 (2000-02-01), pages 278-282, XP002202181, ISSN: 0736-6205
- LASKEN ROGER S ET AL: "Archaebacterial DNA polymerases tightly bind uracil-containing DNA", JOURNAL OF BIOLOGICAL CHEMISTRY, AMERICAN SOCIETY FOR BIOCHEMISTRY AND MOLECULAR BIOLOGY, US, vol. 271, no. 30, 1 January 1996 (1996-01-01), pages 17692-17696, XP002152082, ISSN: 0021-9258, DOI: 10.1074/JBC.271.30.17692

## Description

### BACKGROUND

Non-specific primer extension prior to reaction initiation in thermocycling DNA amplification reactions such as polymerase chain reaction (PCR), or isothermal DNA amplification reactions such as loop-mediated isothermal amplification (LAMP) may inhibit specific product formation, and lead to non-specific amplification and reaction irreproducibility. It is, therefore, desirable to block the activity of the polymerase, and hence primer extension, prior to reaction initiation. This has been achieved using antibodies (Kellogg, et al., Biotechniques, 16(6): 1134-7 (1994)), affybodies (Affibody AB, Stockholm, Sweden), aptamers (Dang, et al., Journal of Molecular Biology, 264(2):268-78 (1996)), and chemical modification of the polymerase (US Patent No. 6,183,998). Although each of these techniques can be effective, they each have unique limitations. For example, preparation of antibodies requires use of animal systems, affybodies and aptamers require screening libraries of molecular variants, and chemical modifications require extra heat incubation steps to reverse the inactivating modification. It would be desirable to have a generalizable approach to rapidly and effectively create hot-start inhibitors targeted towards DNA polymerases. WO0102559 discloses RNA/DNA hybrid hairpins that inhibit a thermostable reverse transcriptase.

### SUMMARY

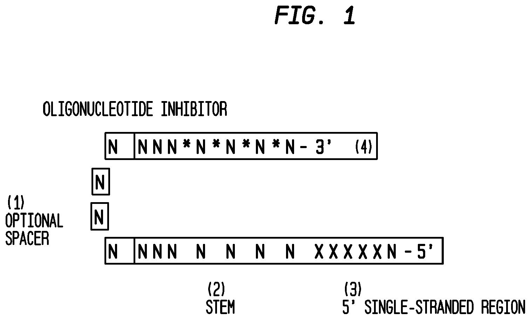

In general, in one aspect, a preparation is provided that includes: a synthetic single-strand nucleic acid having a 3' end and a 5' end, capable of forming a double-stranded region that extends from the 3' end and a single-stranded region having a 5' single-strand extension containing at least one uracil or inosine; and a buffer. An example of a synthetic single-strand nucleic acid is shown in Figure 1.

Embodiments may include one or more of the following features: the at least one double-strand region has a melting temperature (Tm) of at least 10°C less than a Tm for a target DNA in an amplification reaction, for example, below 90°C, 89°C, 88°C, 87°C, 86°C, 85°C, 75°C, 65°C, 55°C, 45°C or 35°C; a uracil or inosine is positioned at the fourth position in the 5' single-strand extension numbered from the 3' end; the synthetic nucleic acid is capable of forming a plurality of single-strand regions; a second single-strand region is a spacer; a third single-strand region forms a single-stranded loop at an internal location in the synthetic nucleic acid; the buffer may contain at least one of a polymerase, dNTPs, or primers; the spacer comprises hexa-ethylene glycol, a 3 carbon molecule or a 1,2-dideoxyribose; the synthetic nucleic acid contains a derivative nucleotide and/or nucleotide linkage in a nucleic acid sequence at the 3' end where the derivative nucleotide may be selected from one or more inverted nucleotides, di-deoxynucleotides or amino-modified nucleotides; for example, the nucleotide linkage may be a phosphorothioate linkage;

In an embodiment, the preparation may additionally include one or more polymerases for example, one or more thermostable polymerases, for example at least one archaeal polymerase; a bacterial polymerase, and/or a variant of a wild type archaeal or bacterial polymerase. The synthetic nucleic acid and the polymerase may be present in a molar ratio of between 0.5:1 to 10:1.

In general in one aspect, a variant of a wild type polymerase includes at least 93% sequence identity to SEQ ID NO:25 and further includes at least one mutation at an amino acid position corresponding to 278, 307, and/or 402 in SEQ ID NO:25. In another aspects, mutations at 278, 307 and/or 402 may be inserted into any of the Bst polymerase variants described in U.S. application serial number 13/823,811.

Embodiments may include one or more of the following features of the preparation: fusion of variant polymerase to a DNA binding domain such as Sso7d; and/or the variant polymerase optionally having an amino acid at one or more of the positions corresponding to 278, 307, and/or 402 that is not a histidine; for example where one or more mutations may be selected from a group of mutations corresponding to H278Q, H307R, H402Q.

In general in one aspect, a method is provided for inhibiting a polymerase extension reaction; that includes adding a preparation described above to a mixture containing a polymerase, a target DNA and dNTPs; and maintaining for a period of time prior to extension or amplification of the target DNA, the mixture at a temperature below the Tm of the double-stranded portion of the synthetic nucleic acid.

Embodiments may include one or more of the following features:
the at least one double-strand region has a Tm of at least 10°C less than a Tm for a target DNA in an amplification reaction, for example, below 90°C, 89°C , 88°C, 87°C, 86°C, 85°C, 75°C, 65°C, 55°C, 45°C or 35°C; a uracil or inosine is positioned at the fourth position in the 5' single-strand extension numbered from the 3'end;
the synthetic nucleic acid may include additional single-stranded nucleic acid regions such as a second single-strand region is a spacer; where for example, the spacer may include a hexa-ethylene glycol, a 3 carbon molecule or a 1,2-dideoxyribose; and/or a third single-strand region forms a single-stranded loop at an internal location in the synthetic nucleic acid.

In an embodiment, the synthetic nucleic acid contains at least one derivative nucleotide and/or nucleotide linkage at the 3' end where the at least one derivative nucleotide may be selected from one or more inverted nucleotides, di-deoxynucleotides or amino-modified nucleotides; and for example, the at least one nucleotide linkage may be a phosphorothioate linkage.

In an embodiment, the one or more polymerases may include one or more thermostable polymerases, for example at least one archaeal polymerase; a bacterial polymerase, and/or a variant of a wild type archaeal or bacterial polymerase; and the synthetic nucleic acid and the polymerase may be present in a molar ratio of between 0.5:1 to 10:1.

In one embodiment, an additional step may be included of reversing the inhibition of the polymerase extension reaction by raising the reaction temperature above a Tm for the synthetic nucleic acid. The invention itself is defined in the appended claims.

### BRIEF DESCRIPTION OF THE DRAWINGS

Figure 1 shows a synthetic nucleic acid in the form of a hairpin oligonucleotide containing a 5' overhang, a 3' blocked end to prevent DNA polymerase extension and exonuclease cleavage and at least one non-standard base. (1) is the optional spacer; (2) is the double-stranded region or "stem"; (3) is the 5' single-strand; and (4) is the blocked 3' end: N = rNMP, dNMP or non-standard base; X = base that is recognized by the DNA polymerase uracil binding pocket; * = 3' end modifications: phosphorothioate bonds and/or inverted base and/or dideoxynucleoside.

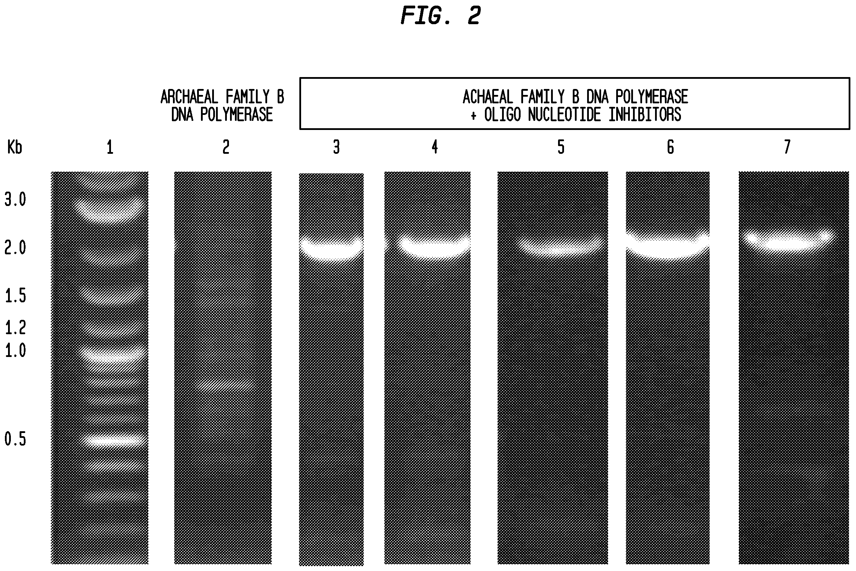

Figure 2 shows a gel of the PCR products obtained with an Archaeal polymerase in the presence or absence of hairpin oligonucleotide inhibitors. In the absence of the hairpin oligonucleotide, the polymerase fails to amplify the expected 2kb product. In the presence of the oligonucleotides the 2kb product is amplified.
Lane 1 contains 2-log DNA ladder (New England Biolabs, Ipswich, MA), a MW marker for detection of 2Kb amplicon.
Lane 2 contains 5nM Archaeal Family B DNA polymerase without the synthetic nucleic acid present.
Lane 3 contains 5nM Archaeal Family B DNA polymerase and 5nM the synthetic nucleic acid, TM39U1G-Is.
Lane 4 contains 5nM Archaeal Family B DNA polymerase and 5nM the synthetic nucleic acid, TM39U1G-I*.
Lane 5 contains 5nM Archaeal Family B DNA polymerase and 5nM the synthetic nucleic acid, TM39U.
Lane 6 contains 5nM Archaeal Family B DNA polymerase and 5nM the synthetic nucleic acid, TM39Loop10T.
Lane 7 contains 5nM Archaeal Family B DNA polymerase and 5nM the synthetic nucleic acid, TM39U3-Is.

### DETAILED DESCRIPTION OF THE EMBODIMENTS

Synthetic nucleic acids are described that reversibly inhibit polymerase extension reactions. These synthetic nucleic acid preferably contain at least one non-standard base (e.g. U or I) in a 5' single-strand overhang adjacent to a double-strand region. If the double-strand region is denatured into a single-strand or strands, the synthetic nucleic acid no longer blocks the polymerase from replicating substrate DNA. Preferably, inhibition of polymerase activity occurs at a first temperature that is at least 10°C lower than a second temperature suitable for polymerase extension reactions. A polymerase extension reaction refers to the extension of a first single-strand nucleic acid by a polymerase where the extension is complementary to a second nucleic acid in association with the first strand.

In an embodiment, a synthetic nucleic acid is engineered so that the double-strand region melts at a desired temperature which is selected to melt at about 15°C or 14°C or 13°C or 12°C or 11°C or 10°C or 9°C or 8°C below polymerization extension conditions. Polymerase extension conditions include conditions for isothermal amplification occurring at for example 65°C or a thermocycling amplification such as PCR which occurs at higher temperatures such as about 95°C. For example, the double-strand region in the synthetic nucleic acid may be designed to remain intact at a specific temperature in the range of - 80°C to 37°C but become denatured at a specific temperature in a range of 37°C to 100°C. The Tm of the synthetic nucleic acid can be modulated by one or more factors that include: changing the sequence or length of the double-strand region, changing the length of an internal single-strand region, adding mismatched or modified bases to the double-strand region, selecting a nucleotide composition having weaker base pairing properties such as an adenine, thymine or uracil rich sequence, or a sequence containing inosine, or abasic sites such as 1',2' dideoxyribose in a polymerization reaction buffer with a selected salt type (for example magnesium) and concentration. An example of a buffer is Thermopol® Buffer (New England Biolabs, Ipswich, MA). The design of a synthetic nucleic acid reversible inhibitor of polymerase extension reactions includes the following features: the synthetic nucleic acid can be DNA, DNA/RNA, RNA, or RNA/RNA; it can be formed from two single-strands or from a single nucleic acid (oligonucleotide) but should be capable of forming at least one double-strand region and a 5' single-strand overhang. It may optionally contain a plurality of single-strand regions and a plurality of double-strand regions. If the synthetic nucleic acid is an oligonucleotide, it should be capable of folding in such a way as to contain at least one double-strand region at a temperature lower than the reaction temperature as described above. The oligonucleotide may have a length in the range of 8-200 nucleotides. Any double-strand region in the inhibitor preferably has a length of 4-35 nucleotides.

The 5' single-strand overhang should be at least 4 nucleotides and preferably less than 100 nucleotides in length, for example 4-40 nucleotides, for example 6-10 nucleotides, and should contain one or more non-standard nucleotides such as U or I positioned between the second and tenth position of the overhang counted from the double-strand region, for example in the fourth position where the one or more non-standard nucleotides may be 1 to 5 uracils or 1 to 5 inosines. For example, the sequences shown in Table 1 were all found to be effective as reversible binding oligonucleotides.

In addition, a synthetic nucleic acid may optionally have a 3' end that is resistant to exonuclease activity and/or non-extendable by a polymerase. The 3' end of the oligonucleotide can be blocked from extension by modification, such as dideoxynucleotides, spacer molecules, inverted bases or amino-modified nucleotides. The 3' end can be made resistant to exonuclease degradation by the addition of phosphorothioate linkages between one or more bases at or near the 3' end or the use of inverted bases at the 3' end. The oligonucleotide can be made non-amplifiable by adding non-replicable bases in the internal sequence, such as carbon spacers, 1',2'-Dideoxyribose, abasic site, or thymine dimers.

Table 1 provides examples of synthetic nucleic acid molecules capable of forming hairpins and that were found to be effective in the assays described herein. The exemplified synthetic nucleic acid molecules have spacers of Tₙ or Xₙ where T₍₄₋₉₎ or X₍₁₋₄₎, a 5' end containing a modified base, U₍₁₋₅₎, or I₍₁₋₃₎ and has a U or an I at position 4 counted from the double-stranded region. The 5' end varies as shown.

**Table 1**

| **Oligonucleotides tested and effective in Hot Start PCR** | |
|---|---|
| Oligo Length | Sequence containing uracil (U) or Inosine (I) *= phosphorothioate bonds |
| 28 | TUUUUUCTATCCTTATTTTTAAGGA*T*A*G (SEQ ID NO:3) |
| 24 | TUUUUUAGCTAGGTTTTCCTA*G*C*T (SEQ ID NO:4) |
| 24 | TUUUUUGCAGCGATTTTTCGC*T*G*C (SEQ ID NO:5) |
| 30 | TUUUUUGAGACTCGRCTTTTGACGAGT*C*T*C (SEQ ID NO:6) |
| 34 | TUUUUUCTATCCTTAACGTTTTCGTTAAGGA*T*A*G (SEQ ID NO:7) |
| 30 | TUUUUUACACTTCCGGTTTTCCGGAAG*T*G*T (SEQ ID NO:8) |
| 31 | TUUUUUCTATCCTTAACGXCGTTAAGGA*T*A*G (SEQ ID NO:9) |
| 34 | TUUUUUCTATCCTTAACGXXXXCGTTAAGGA*T*A*G (SEQ ID NO:10) |
| 36 | TUUUUUCTATCCTTAACGTTTTTTCGTTAAGGA*T*A*G (SEQ ID NO:11) |
| 40 | TUUUUUCTATCCTTAACGTTTTTTTTTTTCGTTAAGGA*T*A*G (SEQ ID NO:12) |
| 34 | TUUUUUCTATCCTTAACITTTTCGTTAAGGA*T*A*G (SEQ ID NO:13) |
| 34 | TUUUUUCTATCCTTAACITTTTCGTTAAGG*A*T*A*G (SEQ ID NO:14) |
| 34 | TUUUUUATCTCCTTAACITTTTCGTTAAGGAGAinvd*T* (SEQ ID NO:15) |
| 34 | TUUUUUCTITCCTTIICGTTTTCGTTAAGGA*T*A*G (SEQ ID NO:16) |
| 34 | TAUGGACTATCCTTAACGTTTTCGTTAAGGA*T*A*G (SEQ ID NO:17) |
| 34 | TUUUGACTATCCTTAACGTTTTCGTTAAGGA*T*A*G (SEQ ID NO:18) |
| 34 | TTITTTCTATCCTTAACGTTTTCGTTAAGGA*T*A*G (SEQ ID NO:19) |
| 34 | TTITTTCTATCCTTAACGTTTTCGRRAAGG*A*T*A*G (SEQ ID NO:20) |
| 34 | TTITTTATCTCCTTAACGTTTTCGRRAAGGAGAinvd*T* (SEQ ID NO:21) |
| 34 | TIIITTCTATCCTTAACGTTTTCGTTAAGGA*T*A*G (SEQ ID NO:22) |
| 34 | TIIITTCTATCCTTAACGTTTTCGTTAAGG*A*T*A*G (SEQ ID NO:23) |
| 34 | TIIITTATCTCCTTAACGTTTTCGTTAAGGAGAinvd*T* (SEQ ID NO:24) |

One or more polymerases may be added to the synthetic nucleic acid. The polymerases may be thermostable polymerases such as wild type or recombinant Archaeal DNA polymerases or bacterial DNA polymerases or variants (mutants) thereof including fusion proteins where the polymerase or variants thereof may be fused to a DNA binding domain such as Sso7d (for example, US Patent No. 7,666,645). A variant of a bacterial polymerase is exemplified at least 90%, 91%, 92% 93%, 95%, or 98% amino acid sequence homology or identity with SEQ ID NO:25 prior to fusion to a DNA binding domain if such is present. Regardless of the presence of an additional DNA binding domain, the variant preferably includes one or more mutations at positions corresponding to 52 (not R), 278, 307, 402, and/or 578 (not R) in SEQ ID NO:25, for example, one or more of the following mutations: H278Q, H307R, H402Q. Additional mutations may be optionally introduced into the polymerase by routine methods of random or directed mutagenesis.

Amplification procedures referred to herein include standard thermocycling or isothermal amplification reactions such as PCR amplification or LAMP (Gill, et al., Nucleos. Nucleot. Nucleic Acids, 27:224-43 (2008); Kim, et al, Bioanalysis, 3:227-39 (2011); Nagamine, et al., Mol. Cel. Probes, 16:223-9 (2002); Notomi, et al., Nucleic Acids Res., 28:E63 (2000); and Nagamine, et al., Clin. Chem., 47:1742-3 (2001)), helicase displacement amplification (HDA), recombinase polymerase amplification (RPA), nicking enzyme amplification reaction (NEAR) and/or strand displacement amplification (SDA). Variant polymerases described herein may be used in amplification or sequencing reactions with or without the use of synthetic nucleic acids described herein.
Amino acid sequence for a wild type Bst polymerase

### EXAMPLE

### Assay to measure inhibition of polymerase activity prior to PCR cycling

Inhibition of polymerase activity was measured at a temperature below that used in the PCR assay which followed.

The assay was performed as follows:
Primers were made for PCR to produce a 2kb Lambda DNA amplicon. Additionally, the 3' end of the reverse primer contained 8 nucleotides that could anneal to Lambda DNA creating a false priming site producing a non-specific 737 bp amplicon.

The PCR assay was done in the presence of high levels of human genomic DNA and the reaction mixture was incubated with the thermostable polymerase at 25°C for 15 minutes prior to PCR cycling. These conditions created many opportunities to form non-specific products. The presence of a nucleic acid composition to inhibit polymerase activity prior to amplification was required to yield a 2kb amplicon, with minimal or no non-specific products. The reaction mix was set up on ice and contained the following reagents: Thermopol Buffer, 0.4 pg/µl Lambda DNA, 2.0 ng/µl Jurkat genomic DNA, 0.2 mM dNTP and 0.2 µM primers.
Forward primer, L30350F: 5'CCTGCTCTGCCGCTTCACGC3' (SEQ ID No:1)
Reverse primer, L2kbalt4rv: 5'GGGCCGTGGCAGTCGCATCCC3' (SEQ ID No:2)

0.25 µl to 0.50 µl of 2.0 units/µl Vent® DNA Polymerase (NEB, Ipswich, MA) with or without the nucleic acid composition (see Figure 2) was added to 25 µl or 50 µl of the reaction mix, and transferred to a PCR machine and cycled at 25°C for 15-30 minutes, then cycled 35 times at 98°C for 10 seconds, 45°C for 20 seconds, 72°C for 60 seconds, 72°C for 4 minutes. DNA products generated by PCR cycling were analyzed by agarose gel electrophoresis.

In the absence of a reversibly inhibiting synthetic nucleic acid, the polymerase failed to yield the expected 2kb Lamda amplicon. Non-specific products including the 737 bp amplicon were observed. In the presence of oligonucleotide inhibitors, a robust yield of the expected 2kb Lambda amplicon was produced with minimal or no non-specific products.

## Claims

1. A preparation, comprising:
(i) a thermostable archaeal polymerase; and
(ii) a synthetic oligonucleotide having a 3' end and a 5' end; and
(iii) a buffer;
wherein the synthetic oligonucleotide is folded to form a hairpin oligonucleotide comprising:
a double-stranded region that extends from the 3' end, and
a 5' single-strand extension containing at least one uracil or inosine, wherein a uracil or inosine is positioned at the fourth position in the 5' single-strand extension numbered from the 3' end of the 5' single-strand extension;
wherein the synthetic oligonucleotide contains at least one derivative nucleotide and/or phosphorothioate linkage in a nucleic acid sequence at the 3' end, wherein the at least one derivative nucleotide is selected from one or more inverted nucleotides, di-deoxynucleotides or amino-modified nucleotides; and
wherein the double-stranded region of the synthetic oligonucleotide melts at a temperature that is about 15°C below a temperature suitable for polymerization extension.

2. A preparation according to Claim 1, wherein the synthetic oligonucleotide comprises a plurality of single-strand regions.

3. A preparation according to Claim 2, wherein the synthetic oligonucleotide comprises a single-strand spacer region.

4. A preparation according to Claim 3, wherein the spacer comprises hexa-ethylene glycol, a 3 carbon molecule or a 1',2'-dideoxyribose.

5. A preparation according to any of Claims 2-4, wherein the synthetic oligonucleotide comprises a single-stranded loop at an internal location in the synthetic oligonucleotide.

6. A preparation according to any of Claims 1-5, further comprising: at least one of dNTPs, or primers.

7. A preparation according to any of Claims 1-6, wherein the synthetic oligonucleotide and the thermostable archaeal polymerase are present in a molar ratio of between 0.5:1 to 10:1.

8. A method of inhibiting a polymerase extension reaction; comprising:
(a) adding a preparation comprising:
(i) a synthetic oligonucleotide having a 3' end and a 5' end; and
(ii) a buffer;
wherein the synthetic oligonucleotide is folded to form a hairpin oligonucleotide comprising: a double-stranded region that extends from the 3' end, and a 5' single-strand extension containing at least one uracil or inosine, wherein a uracil or inosine is positioned at the fourth position in the 5' single-strand extension numbered from the 3' end of the 5' single-strand extension;
wherein the synthetic oligonucleotide contains at least one derivative nucleotide and/or phosphorothioate linkage in a nucleic acid sequence at the 3' end, wherein the at least one derivative nucleotide is selected from one or more inverted nucleotides, di-deoxynucleotides or amino-modified nucleotides; and
wherein the double-stranded region of the synthetic oligonucleotide melts at a temperature that is about 15°C below a temperature suitable for polymerization extension; to a mixture containing at least one thermostable archaeal polymerase, a target DNA and dNTPs; and
(b) maintaining the mixture at a temperature below the melting temperature (Tm) of the double-stranded portion of the synthetic oligonucleotide prior to extension or amplification of the target DNA.

9. A method according to Claim 8, wherein the synthetic oligonucleotide comprises a plurality of single-strand regions.

10. A method according to Claim 9, wherein the synthetic oligonucleotide comprises a single-strand spacer region.

11. A method according to Claim 10, wherein the spacer comprises hexa-ethylene glycol, a 3 carbon molecule or a 1',2'-dideoxyribose.

12. A method according to any of Claims 8-11, wherein the synthetic oligonucleotide comprises a single-stranded loop at an internal location in the synthetic nucleic acid.

13. The method according to any of Claims 8-12, further comprising: reversing the inhibition of the polymerase extension reaction by raising the reaction temperature above the melting temperature (Tm) for the synthetic oligonucleotide.

## Patentansprüche

1. Aufbereitung, umfassend:
(i) eine thermostabile archäische Polymerase; und
(ii) ein synthetisches Oligonukleotid mit einem 3'-Ende und einem 5'-Ende; und
(iii) einem Puffer;
wobei das synthetische Oligonukleotid zur Bildung eines Haarnadel-Oligonukleotids gefaltet ist, umfassend:
eine Doppelstrangregion, die sich vom 3'-Ende erstreckt, und
eine 5'-Einzelstrangverlängerung, enthaltend mindestens ein Uracil oder Inosin, wobei ein Uracil oder Inosin an Position vier in der 5'-Einzelstrangverlängerung, nummeriert vom 3'-Ende der 5'-Einzelstrangverlängerung, positioniert ist;
wobei das synthetische Oligonukleotid mindestens ein Nukleotidderivat und/oder eine Phosphorthioatverknüpfung in einer Nukleinsäuresequenz am 3'-Ende enthält, wobei das mindestens eine Nukleotidderivat aus einem oder mehr invertierten Nukleotid(en), Didesoxynukleotid(en) oder Aminomodifizierten Nukleotid(en) ausgewählt ist; und
wobei die Doppelstrangregion des synthetischen Oligonukleotids bei einer Temperatur schmilzt, die ca. 15 °C unter einer für die Polymerisationsverlängerung geeigneten Temperatur liegt.

2. Aufbereitung nach Anspruch 1, wobei das synthetische Oligonukleotid eine Vielzahl von Einzelstrangregionen umfasst.

3. Aufbereitung nach Anspruch 2, wobei das synthetische Oligonukleotid eine Einzelstrang-Spacerregion umfasst.

4. Aufbereitung nach Anspruch 3, wobei der Spacer Hexaethylenglycol, ein 3-Kohlenstoffmolekül oder eine 1',2'-Didesoxyribose umfasst.

5. Aufbereitung nach einem der Ansprüche 2 bis 4, wobei das synthetische Oligonukleotid eine Einzelstrangschleife an einem internen Ort im synthetischen Oligonukleotid umfasst.

6. Aufbereitung nach einem der Ansprüche 1 bis 5, weiter umfassend: mindestens eines von dNTPs oder einen von Primern.

7. Aufbereitung nach einem der Ansprüche 1 bis 6, wobei das synthetische Oligonukleotid und die thermostabile archäische Polymerase in einem Molverhältnis zwischen 0,5:1 und 10:1 vorliegen.

8. Verfahren zum Inhibieren einer Polymerase-Verlängerungsreaktion; umfassend:
(a) Zufügen einer Aufbereitung, umfassend:
(i) ein synthetisches Oligonukleotid mit einem 3'-Ende und einem 5'-Ende; und
(ii) einen Puffer;
wobei das synthetische Oligonukleotid zur Bildung eines Haarnadel-Oligonukleotids gefaltet ist, umfassend: eine Doppelstrangregion, die sich vom 3'-Ende erstreckt, und eine 5'-Einzelstrangverlängerung, enthaltend mindestens ein Uracil oder Inosin, wobei ein Uracil oder Inosin an Position vier in der 5'-Einzelstrangverlängerung, nummeriert vom 3'-Ende der 5'-Einzelstrangverlängerung, positioniert ist;
wobei das synthetische Oligonukleotid mindestens ein Nukleotidderivat und/oder eine Phosphorthioatverknüpfung in einer Nukleinsäuresequenz am 3'-Ende enthält, wobei das mindestens eine Nukleotidderivat aus einem oder mehr invertierten Nukleotid(en), Didesoxynukleotid(en) oder Aminomodifizierten Nukleotid(en) ausgewählt ist; und
wobei die Doppelstrangregion des synthetischen Oligonukleotids bei einer Temperatur schmilzt, die ca. 15 °C unter einer für die Polymerisationsverlängerung geeigneten Temperatur liegt;
zu einem Gemisch, das mindestens eine thermostabile archäische Polymerase, eine Target-DNA und dNTPs enthält; und
(b) Aufrechterhalten des Gemischs bei einer Temperatur unter der Schmelztemperatur (Tm) des Doppelstranganteils des synthetischen Oligonukleotids vor der Verlängerung oder Amplifikation der Target-DNA.

9. Verfahren nach Anspruch 8, wobei das synthetische Oligonukleotid eine Vielzahl von Einzelstrangregionen umfasst.

10. Verfahren nach Anspruch 9, wobei das synthetische Oligonukleotid eine Einzelstrang-Spacerregion umfasst.

11. Verfahren nach Anspruch 10, wobei der Spacer Hexaethylenglycol, ein 3-Kohlenstoffmolekül oder eine 1',2'-Didesoxyribose umfasst.

12. Verfahren nach einem der Ansprüche 8 bis 11, wobei das synthetische Oligonukleotid eine Einzelstrangschleife an einem internen Ort in der synthetischen Nukleinsäure umfasst.

13. Verfahren nach einem der Ansprüche 8 bis 12, weiter umfassend die Reversion der Inhibition der Polymerase-Verlängerungsreaktion durch Erhöhen der Reaktionstemperatur über die Schmelztemperatur (Tm) für das synthetische Oligonukleotid.

## Revendications

1. Préparation, comprenant :
(i) une polymérase thermostable issue d'une archée ; et
(ii) un oligonucléotide de synthèse comportant une extrémité 3' et une extrémité 5' ; et
(iii) un tampon ;
dans laquelle l'oligonucléotide de synthèse est replié pour former un oligonucléotide en épingle à cheveux comprenant :
une région bicaténaire qui s'étend à partir de l'extrémité 3', et
une extension monocaténaire 5' contenant au moins un uracile ou une inosine, un uracile ou une inosine étant positionné(e) à la quatrième position dans l'extension monocaténaire 5' numérotée à partir de l'extrémité 3' de l'extension monocaténaire 5' ;
dans laquelle l'oligonucléotide de synthèse contient au moins un nucléotide dérivé et/ou une liaison phosphorothioate dans une séquence d'acide nucléique à l'extrémité 3', ledit au moins un nucléotide dérivé étant sélectionné parmi un ou plusieurs nucléotides inversés, didésoxynucléotides ou nucléotides comportant une modification amino ; et
dans laquelle la région bicaténaire de l'oligonucléotide de synthèse fond à une température qui est d'environ 15 °C inférieure à une température convenant pour l'extension/polymérisation.

2. Préparation selon la revendication 1, dans laquelle l'oligonucléotide de synthèse comprend une pluralité de régions monocaténaires.

3. Préparation selon la revendication 2, dans laquelle l'oligonucléotide de synthèse comprend une région d'espacement monocaténaire.

4. Préparation selon la revendication 3, dans laquelle la région d'espacement comprend de l'hexaéthylène glycol, une molécule comportant 3 atomes de carbone, ou un 1',2'-didésoxyribose.

5. Préparation selon l'une quelconque des revendications 2 à 4, dans laquelle l'oligonucléotide de synthèse comprend une boucle monocaténaire à un emplacement interne dans l'oligonucléotide de synthèse.

6. Préparation selon l'une quelconque des revendications 1 à 5, comprenant en outre au moins l'un de dNTP ou d'amorces.

7. Préparation selon l'une quelconque des revendications 1 à 6, dans laquelle l'oligonucléotide de synthèse et la polymérase thermostable issue d'une archée sont présents selon un rapport molaire de 0,5:1 à 10:1.

8. Procédé d'inhibition d'une réaction d'extension par la polymérase, comprenant :
(a) l'addition d'une préparation comprenant :
(i) un oligonucléotide de synthèse comportant une extrémité 3' et une extrémité 5' ; et
(ii) un tampon ;
dans lequel l'oligonucléotide de synthèse est replié pour former un oligonucléotide en épingle à cheveux comprenant : une région bicaténaire qui s'étend à partir de l'extrémité 3', et une extension monocaténaire 5' contenant au moins un uracile ou une inosine, un uracile ou une inosine étant positionné(e) à la quatrième position dans l'extension monocaténaire 5' numérotée à partir de l'extrémité 3' de l'extension monocaténaire 5' ;
dans lequel l'oligonucléotide de synthèse contient au moins un nucléotide dérivé et/ou une liaison phosphorothioate dans une séquence d'acide nucléique à l'extrémité 3', ledit au moins un nucléotide dérivé étant sélectionné parmi un ou plusieurs nucléotides inversés, didésoxynucléotides ou nucléotides comportant une modification amino ; et
dans lequel la région bicaténaire de l'oligonucléotide de synthèse fond à une température qui est d'environ 15 °C inférieure à une température convenant pour l'extension/polymérisation ;
à un mélange contenant au moins une polymérase thermostable issue d'une archée, un ADN cible et des dNTP ; et
(b) le maintien du mélange à une température inférieure à la température de fusion (Tₘ) de la partie bicaténaire de l'oligonucléotide de synthèse avant l'extension ou l'amplification de l'ADN cible.

9. Procédé selon la revendication 8, dans lequel l'oligonucléotide de synthèse comprend une pluralité de régions monocaténaires.

10. Procédé selon la revendication 9, dans lequel l'oligonucléotide de synthèse comprend une région d'espacement monocaténaire.

11. Procédé selon la revendication 10, dans lequel la région d'espacement comprend de l'hexaéthylène glycol, une molécule comportant 3 atomes de carbone, ou un 1',2'-didésoxyribose.

12. Procédé selon l'une quelconque des revendications 8 à 11, dans lequel l'oligonucléotide de synthèse comprend une boucle monocaténaire à un emplacement interne dans l'acide nucléique de synthèse.

13. Procédé selon l'une quelconque des revendications 8 à 12, comprenant en outre : l'inversion de l'inhibition de la réaction d'extension par la polymérase en augmentant la température de réaction jusqu'à une température supérieure à la température de fusion (Tₘ) pour l'oligonucléotide de synthèse.


### SEQUENCE LISTING

<110> New England Biolabs, Inc.
<120> Synthetic Nucleic Acids for Polymerization Reactions
<130> NEB-344-PCT
<150> 61/623,110
   <151> 2012-04-12
<160> 25
<170> PatentIn version 3.5
<210> 1
   <211> 20
   <212> DNA
   <213> Artificial Sequence
<220>
   <223> Primer
<400> 1
   cctgctctgc cgcttcacgc 20
<210> 2
   <211> 21
   <212> DNA
   <213> Artificial Sequence
<220>
   <223> Primer
<400> 2
   gggccgtggc agtcgcatcc c 21
<210> 3
   <211> 28
   <212> DNA
   <213> Artificial Sequence
<220>
   <223> Synthetic construct
<220>
   <221> modified_base
   <222> (26)..(26)
   <223> phosphorothioate bond
<220>
   <221> modified_base
   <222> (27)..(27)
   <223> phosphorothioate bond
<220>
   <221> modified_base
   <222> (28)..(28)
   <223> phosphorothioate bond
<400> 3
   tuuuuuctat ccttattttt aaggatag 28
<210> 4
   <211> 24
   <212> DNA
   <213> Artificial Sequence
<220>
   <223> Synthetic construct
<220>
   <221> modified_base
   <222> (22)..(22)
   <223> phosphorothioate bond
<220>
   <221> modified_base
   <222> (23)..(23)
   <223> phosphorothioate bond
<220>
   <221> modified_base
   <222> (24)..(24)
   <223> phosphorothioate bond
<400> 4
   tuuuuuagct aggttttcct agct 24
<210> 5
   <211> 24
   <212> DNA
   <213> Artificial Sequence
<220>
   <223> Synthetic construct
<220>
   <221> modified_base
   <222> (22)..(22)
   <223> phosphorothioate bond
<220>
   <221> modified_base
   <222> (23)..(23)
   <223> phosphorothioate bond
<220>
   <221> modified_base
   <222> (24)..(24)
   <223> phosphorothioate bond
<400> 5
   tuuuuugcag cgatttttcg ctgc 24
<210> 6
   <211> 30
   <212> DNA
   <213> Artificial Sequence
<220>
   <223> Synthetic Construct
<220>
   <221> modified_base
   <222> (28)..(28)
   <223> phosphorothioate bond
<220>
   <221> modified_base
   <222> (29)..(29)
   <223> phosphorothioate bond
<220>
   <221> modified_base
   <222> (30)..(30)
   <223> phosphorothioate bond
<400> 6
   tuuuuugaga ctcgrctttt gacgagtctc 30
<210> 7
   <211> 34
   <212> DNA
   <213> Artificial Sequence
<220>
   <223> Synthetic construct
<220>
   <221> modified_base
   <222> (32)..(32)
   <223> phosphorothioate bond
<220>
   <221> modified_base
   <222> (33)..(33)
   <223> phosphorothioate bond
<220>
   <221> modified_base
   <222> (34)..(34)
   <223> phosphorothioate bond
<400> 7
   tuuuuuctat ccttaacgtt ttcgttaagg atag 34
<210> 8
   <211> 30
   <212> DNA
   <213> Artificial Sequence
<220>
   <223> Synthetic construct
<220>
   <221> modified_base
   <222> (28)..(28)
   <223> phosphorothioate bond
<220>
   <221> modified_base
   <222> (29)..(29)
   <223> phosphorothioate bond
<220>
   <221> modified_base
   <222> (30)..(30)
   <223> phosphorothioate bond
<400> 8
   tuuuuuacac ttccggtttt ccggaagtct 30
<210> 9
   <211> 31
   <212> DNA
   <213> Artificial Sequence
<220>
   <223> Synthetic construct
<220>
   <221> misc_feature
   <222> (19)..(19)
   <223> n represents any nucleotide
<220>
   <221> modified_base
   <222> (29)..(29)
   <223> phosphorothioate bonds
<220>
   <221> modified_base
   <222> (30)..(30)
   <223> phosphorothioate bonds
<220>
   <221> modified_base
   <222> (31)..(31)
   <223> phosphorothioate bonds
<400> 9
   tuuuuuctat ccttaacgnc gttaaggata g 31
<210> 10
   <211> 34
   <212> DNA
   <213> Artificial Sequence
<220>
   <223> Synthetic construct
<220>
   <221> misc_feature
   <222> (19)..(22)
   <223> n represents any nucleotide
<220>
   <221> modified_base
   <222> (32)..(32)
   <223> phosphorothioate bond
<220>
   <221> modified_base
   <222> (33)..(33)
   <223> phosphorothioate bond
<220>
   <221> modified_base
   <222> (34)..(34)
   <223> phosphorothioate bond
<400> 10
   tuuuuuctat ccttaacgnn nncgttaagg atag 34
<210> 11
   <211> 36
   <212> DNA
   <213> Artificial Sequence
<220>
   <223> Synthetic construct
<220>
   <221> modified_base
   <222> (34)..(34)
   <223> phosphorothioate bond
<220>
   <221> modified_base
   <222> (35)..(35)
   <223> phosphorothioate bond
<220>
   <221> modified_base
   <222> (36)..(36)
   <223> phosphorothioate bond
<400> 11
   tuuuuuctat ccttaacgtt ttttcgttaa ggatag 36
<210> 12
   <211> 40
   <212> DNA
   <213> Artificial Sequence
<220>
   <223> Synthetic construct
<220>
   <221> modified_base
   <222> (38)..(38)
   <223> phosphorothioate bond
<220>
   <221> modified_base
   <222> (39)..(39)
   <223> phosphorothioate bond
<220>
   <221> modified_base
   <222> (40)..(40)
   <223> phosphorothioate bond
<400> 12
   tuuuuuctat ccttaacgtt ttttttttcg ttaaggatag 40
<210> 13
   <211> 34
   <212> DNA
   <213> Artificial Sequence
<220>
   <223> Synthetic construct
<220>
   <221> misc_feature
   <222> (18)..(18)
   <223> n represents inosine
<220>
   <221> modified_base
   <222> (32)..(32)
   <223> phosphorothioate bond
<220>
   <221> modified_base
   <222> (33)..(33)
   <223> phosphorothioate bond
<220>
   <221> modified_base
   <222> (34)..(34)
   <223> phosphorothioate bond
<400> 13
   tuuuuuctat ccttaacntt ttcgttaagg atag 34
<210> 14
   <211> 34
   <212> DNA
   <213> Artificial Sequence
<220>
   <223> Synthetic construct
<220>
   <221> misc_feature
   <222> (18)..(18)
   <223> n represents inosine
<220>
   <221> modified_base
   <222> (31)..(31)
   <223> phosphorothioate bond
<220>
   <221> modified_base
   <222> (32)..(32)
   <223> phosphorothioate bond
<220>
   <221> modified_base
   <222> (33)..(33)
   <223> phosphorothioate bond
<220>
   <221> modified_base
   <222> (34)..(34)
   <223> phosphorothioate bond
<400> 14
   tuuuuuctat ccttaacntt ttcgttaagg atag 34
<210> 15
   <211> 34
   <212> DNA
   <213> Artificial Sequence
<220>
   <223> Synthetic construct
<220>
   <221> misc_feature
   <222> (18)..(18)
   <223> n represents inosine
<220>
   <221> misc_feature
   <222> (34)..(34)
   <223> n represents inosine
<400> 15
   tuuuuuatct ccttaacntt ttcgttaagg agat 34
<210> 16
   <211> 34
   <212> DNA
   <213> Artificial Sequence
<220>
   <223> Synthetic construct
<220>
   <221> misc_feature
   <222> (9)..(9)
   <223> n represents inosine
<220>
   <221> misc_feature
   <222> (15)..(16)
   <223> x represents inosine
<220>
   <221> modified_base
   <222> (32)..(32)
   <223> phosphorothioate bond
<220>
   <221> modified_base
   <222> (33)..(33)
   <223> phosphorothioate bond
<220>
   <221> modified_base
   <222> (34)..(34)
   <223> phosphorothioate bond
<400> 16
   tuuuuuctnt ccttnncgtt ttcgttaagg atag 34
<210> 17
   <211> 34
   <212> DNA
   <213> Artificial Sequence
<220>
   <223> Synthetic construct
<220>
   <221> modified_base
   <222> (32)..(32)
   <223> phosphorothioate bond
<220>
   <221> modified_base
   <222> (33)..(33)
   <223> phosphorothioate bond
<220>
   <221> modified_base
   <222> (34)..(34)
   <223> phosphorothioate bond
<400> 17
   tauggactat ccttaacgtt ttcgttaagg atag 34
<210> 18
   <211> 34
   <212> DNA
   <213> Artificial Sequence
<220>
   <223> Synthetic construct
<220>
   <221> modified_base
   <222> (32)..(32)
   <223> phosphorothioate bond
<220>
   <221> modified_base
   <222> (33)..(33)
   <223> phosphorothioate bond
<220>
   <221> modified_base
   <222> (34)..(34)
   <223> phosphorothioate bond
<400> 18
   tuuugactat ccttaacgtt ttcgttaagg atag 34
<210> 19
   <211> 34
   <212> DNA
   <213> Artificial Sequence
<220>
   <223> Synthetic construct
<220>
   <221> misc_feature
   <222> (3)..(3)
   <223> n represents inosine
<220>
   <221> modified_base
   <222> (32)..(32)
   <223> phosphorothioate bond
<220>
   <221> modified_base
   <222> (33)..(33)
   <223> phosphorothioate bond
<220>
   <221> modified_base
   <222> (34)..(34)
   <223> phosphorothioate bond
<400> 19
   ttntttctat ccttaacgtt ttcgttaagg atag 34
<210> 20
   <211> 34
   <212> DNA
   <213> Artificial Sequence
<220>
   <223> Synthetic construct
<220>
   <221> misc_feature
   <222> (3)..(3)
   <223> n represents inosine
<220>
   <221> modified_base
   <222> (31)..(31)
   <223> phosphorothioate bond
<220>
   <221> modified_base
   <222> (32)..(32)
   <223> phosphorothioate bond
<220>
   <221> modified_base
   <222> (33)..(33)
   <223> phosphorothioate bond
<220>
   <221> modified_base
   <222> (34)..(34)
   <223> phosphorothioate bond
<400> 20
   ttntttctat ccttaacgtt ttcgttaagg atag 34
<210> 21
   <211> 34
   <212> DNA
   <213> Artificial Sequence
<220>
   <223> Synthetic construct
<220>
   <221> misc_feature
   <222> (3)..(3)
   <223> n represents inosine
<220>
   <221> modified_base
   <222> (34)..(34)
   <223> inverted thymine
<400> 21
   ttntttatct ccttaacgtt ttcgttaagg agat 34
<210> 22
   <211> 34
   <212> DNA
   <213> Artificial Sequence
<220>
   <223> Synthetic construct
<220>
   <221> misc_feature
   <222> (2)..(4)
   <223> x represents inosine
<220>
   <221> modified_base
   <222> (32)..(32)
   <223> phosphorothioate bond
<220>
   <221> modified_base
   <222> (33)..(33)
   <223> phosphorothioate bond
<220>
   <221> modified_base
   <222> (34)..(34)
   <223> phosphorothioate bond
<400> 22
   tnnnttctat ccttaacgtt ttcgttaagg atag 34
<210> 23
   <211> 34
   <212> DNA
   <213> Artificial Sequence
<220>
   <223> Synthetic construct
<220>
   <221> misc_feature
   <222> (2)..(4)
   <223> n represents inosine
<220>
   <221> modified_base
   <222> (31)..(31)
   <223> phosphorothioate bond
<220>
   <221> modified_base
   <222> (32)..(32)
   <223> phosphorothioate bond
<220>
   <221> modified_base
   <222> (33)..(33)
   <223> phosphorothioate bond
<220>
   <221> modified_base
   <222> (34)..(34)
   <223> phosphorothioate bond
<400> 23
   tnnnttctat ccttaacgtt ttcgttaagg atag 34
<210> 24
   <211> 34
   <212> DNA
   <213> Artificial Sequence
<220>
   <223> Synthetic construct
<220>
   <221> misc_feature
   <222> (2)..(4)
   <223> n represents inosine
<220>
   <221> modified_base
   <222> (34)..(34)
   <223> inverted thymine
<400> 24
   tnnnttatct ccttaacgtt ttcgttaagg agat 34
<210> 25
   <211> 587
   <212> PRT
   <213> Bacillus stearothermophilus
<400> 25
   Ala Glu Gly Glu Lys Pro Leu Glu Glu Met Glu Phe Ala Ile Val Asp
   1               5                   10                  15
   Val Ile Thr Glu Glu Met Leu Ala Asp Lys Ala Ala Leu Val Val Glu
               20                  25                  30
   Val Met Glu Glu Asn Tyr His Asp Ala Pro Ile Val Gly Ile Ala Leu
           35                  40                  45
   Val Asn Glu His Gly Arg Phe Phe Met Arg Pro Glu Thr Ala Leu Ala
       50                  55                  60
   Asp Ser Gln Phe Leu Ala Trp Leu Ala Asp Glu Thr Lys Lys Lys Ser
   65                  70                  75                  80
   Met Phe Asp Ala Lys Arg Ala Val Val Ala Leu Lys Trp Lys Gly Ile
                   85                  90                  95
   Glu Leu Arg Gly Val Ala Phe Asp Leu Leu Leu Ala Ala Tyr Leu Leu
               100                 105                 110
   Asn Pro Ala Gln Asp Ala Gly Asp Ile Ala Ala Val Ala Lys Met Lys
           115                 120                 125
   Gln Tyr Glu Ala Val Arg Ser Asp Glu Ala Val Tyr Gly Lys Gly Val
       130                 135                 140
   Lys Arg Ser Leu Pro Asp Glu Gln Thr Leu Ala Glu His Leu Val Arg
   145                 150                 155                 160
   Lys Ala Ala Ala Ile Trp Ala Leu Glu Gln Pro Phe Met Asp Asp Leu
                   165                 170                 175
   Arg Asn Asn Glu Gln Asp Gln Leu Leu Thr Lys Leu Glu Gln Pro Leu
               180                 185                 190
   Ala Ala Ile Leu Ala Glu Met Glu Phe Thr Gly Val Asn Val Asp Thr
           195                 200                 205
   Lys Arg Leu Glu Gln Met Gly Ser Glu Leu Ala Glu Gln Leu Arg Ala
       210                 215                 220
   Ile Glu Gln Arg Ile Tyr Glu Leu Ala Gly Gln Glu Phe Asn Ile Asn
   225                 230                 235                 240
   Ser Pro Lys Gln Leu Gly Val Ile Leu Phe Glu Lys Leu Gln Leu Pro
                   245                 250                 255
   Val Leu Lys Lys Thr Lys Thr Gly Tyr Ser Thr Ser Ala Asp Val Leu
               260                 265                 270
   Glu Lys Leu Ala Pro His His Glu Ile Val Glu Asn Ile Leu His Tyr
           275                 280                 285
   Arg Gln Leu Gly Lys Leu Gln Ser Thr Tyr Ile Glu Gly Leu Leu Lys
       290                 295                 300
   Val Val Arg Pro Asp Thr Gly Lys Val His Thr Met Phe Asn Gln Ala
   305                 310                 315                 320
   Leu Thr Gln Thr Gly Arg Leu Ser Ser Ala Glu Pro Asn Leu Gln Asn
                   325                 330                 335
   Ile Pro Ile Arg Leu Glu Glu Gly Arg Lys Ile Arg Gln Ala Phe Val
               340                 345                 350
   Pro Ser Glu Pro Asp Trp Leu Ile Phe Ala Ala Asp Tyr Ser Gln Ile
           355                 360                 365
   Glu Leu Arg Val Leu Ala His Ile Ala Asp Asp Asp Asn Leu Ile Glu
       370                 375                 380
   Ala Phe Gln Arg Asp Leu Asp Ile His Thr Lys Thr Ala Met Asp Ile
   385                 390                 395                 400
   Phe His Val Ser Glu Glu Glu Val Thr Ala Asn Met Arg Arg Gln Ala
                   405                 410                 415
   Lys Ala Val Asn Phe Gly Ile Val Tyr Gly Ile Ser Asp Tyr Gly Leu
               420                 425                 430
   Ala Gln Asn Leu Asn Ile Thr Arg Lys Glu Ala Ala Glu Phe Ile Glu
           435                 440                 445
   Arg Tyr Phe Ala Ser Phe Pro Gly Val Lys Gln Tyr Met Glu Asn Ile
       450                 455                 460
   Val Gln Glu Ala Lys Gln Lys Gly Tyr Val Thr Thr Leu Leu His Arg
   465                 470                 475                 480
   Arg Arg Tyr Leu Pro Asp Ile Thr Ser Arg Asn Phe Asn Val Arg Ser
                   485                 490                 495
   Phe Ala Glu Arg Thr Ala Met Asn Thr Pro Ile Gln Gly Ser Ala Ala
               500                 505                 510
   Asp Ile Ile Lys Lys Ala Met Ile Asp Leu Ala Ala Arg Leu Lys Glu
           515                 520                 525
   Glu Gln Leu Gln Ala Arg Leu Leu Leu Gln Val His Asp Glu Leu Ile
       530                 535                 540
   Leu Glu Ala Pro Lys Glu Glu Ile Glu Arg Leu Cys Glu Leu Val Pro
   545                 550                 555                 560
   Glu Val Met Glu Gln Ala Val Thr Leu Arg Val Pro Leu Lys Val Asp
                   565                 570                 575
   Tyr His Tyr Gly Pro Thr Trp Tyr Asp Ala Lys
               580                 585